# EUROPÄISCHE PATENTSCHRIFT

(11) **EP 0 706 556 B1**
(45) Veröffentlichungstag und Bekanntmachung des Hinweises auf die Patenterteilung: **25.03.1998**
(21) Anmeldenummer: 94919643.0
(22) Anmeldetag: 20.06.1994
(51) Int. Cl.: C11D 3/00, C11D 1/835, A61K 7/48, A01N 33/04

(54) **REINIGUNGS- UND DESINFEKTIONSMITTEL**
CLEANING AND DISINFECTING AGENT
NETTOYANT DESINFECTANT

(30) Priorität: 28.06.1993 FR 9308097
(43) Veröffentlichungstag der Anmeldung: 17.04.1996
(73) Patentinhaber: Henkel-Ecolab GmbH & Co. OHG, 40554 Düsseldorf (DE)
(72) Erfinder: PINOTEAU, Michel, F-06000 Nice (FR)
(74) Vertreter: Hase, Christian, Dr.
(86) Internationale Anmeldenummer: EP9402002
(87) Internationale Veröffentlichungsnummer: WO9500613

(56) Entgegenhaltungen:
- EP-A- 0 333 143
- GB-A- 2 025 229
- GB-A- 2 187 097
- US-A- 3 296 145
- US-A- 3 717 579
- DATABASE WPI Week 9035, Derwent Publications Ltd., London, GB; AN 90-264444 & JP,A,2 184 609 (SANYO CHEM IND LTD) 19. Juli 1990

## Beschreibung

Die vorliegende Erfindung bezieht sich auf ein Reinigungs- und Desinfektionsmittel für die Benutzung zum Desinfizieren der Böden und Wände, insbesondere in Krankenhäusern oder für die antiseptische Behandlung der gesunden Haut.

Nosokomialinfektionen stellen aufgrund ihrer Häufigkeit, ihres potentiellen Schweregrads sowie ihrer wirtschaftlichen und sozialen Folgen ein großes Problem für das öffentliche Gesundheitswesen dar. Sie können durch die Anwendung effizienter Maßnahmen eingedämmt werden, beispielsweise durch die Beachtung strenger Hygiene- und Desinfektionsvorschriften in unterschiedlichen Bereichen, wie etwa bei den Böden, Wänden und Oberflächen aller Art sowie bei den medizinisch-chirurgischen Instrumenten und Geräten.

Der französische Normenausschuß AFNOR definiert die Desinfektion als einen Vorgang mit kurzzeitigem Ergebnis, der die Beseitigung oder Abtötung von Mikroorganismen und/oder die Inaktivierung unerwünschter Viren ermöglicht, die in kontaminierten, neutralen Umgebungen vorhanden sind.

Die zur Zeit für die Desinfektion in Krankenhäusern am häufigsten benutzten Wirksubstanzen sind Aldehyde, quartäre Ammoniumverbindungen und Phenole. Die üblicherweise bei der Desinfektion benutzten Aldehyde sind Formaldehyd und Glutaraldehyd. Sie haben zwar ein besonders breites Wirkungsspektrum, besitzen jedoch eine beträchtliche Toxizität für Benutzer und Patienten.

Das Einatmen von Formaldehyd verursacht beim Menschen ab 0,1 ppm Tränenfluß, bei 1 bis 5 ppm Reizungen der oberen Atemwege mit Nasen-, Rachen- und Kehlkopfschleimhautentzündungen und bei 50 ppm Lungenödeme. Im Krankenhaus hat die Verwendung größerer Mengen Formaldehyd in anatomischpathologischen Labors und in Hämodialyseeinheiten akute Vergiftungserscheinungen verursacht. Außerdem können bei bestimmten Personen, die Formaldehyd in einer Konzentration ab 0,4 ppm ausgesetzt sind, Augenreizungen auftreten. Bei lokaler Einwirkung auf die Schleimhäute kann der Kontakt mit Formaldehyd Reizungen, Veränderungen der Hornhaut oder ekzemartige Läsionen zur Folge haben. Glutaraldehyd kann beim Menschen nach längerem Einatmen Reizungen der Atmungsorgane in Verbindung mit Hustenanfällen verursachen. Bei lokaler Einwirkung auf die Schleimhäute bewirkt Glutaraldehyd Reizungen, Erytheme oder Veränderungen der Hornhaut.

Quartäre Ammoniumverbindungen werden wegen ihrer bakteriziden und reinigenden Eigenschaften häufig als Desinfektionsmittel in Krankenhäusern verwendet. Beim Menschen sind jedoch zahlreiche Fälle von Hauterkrankungen mit Kontaktallergien festgestellt worden. Bei Benutzern, die wiederholt oder über längere Zeiträume Produkte auf der Basis von quartärem Ammonium verwendet hatten, traten Überempfindlichkeitsreaktionen auf. Das Einatmen von Nebeln oder Aerosolen, die quartäre Ammoniumsalze enthalten, kann Lungenödeme zur Folge haben. Von daher ergibt sich die Notwendigkeit, über ein nicht toxisches Reinigungs- und Desinfektionsmittel zu verfügen, das in Krankenhäusern anstelle von Aldehyden und quartären Ammoniumverbindungen verwendet werden kann.

EP-A-333 143 beschreibt flüssige biozide Reinigungsmittel, bestehend aus einem tertiären Alkylamin und einer anionischen Reinigungskomponente. US-A-3 296 145 erwähnt, daß sich Aminoxide als Tenside durch ihre Hautfreundlichkeit auszeichnen. In GB-A-2 187 097 werden Aminoxide als antimikrobielle Wirkstoffe für saure wäßrig-alkoholische Lösungen genannt.

Der Zweck der Erfindung besteht in der Bereitstellung eines neuen Reinigungs- und Desinfektionsmittels, das keine für den Benutzer toxischen Eigenschaften besitzt und das sich einfach zur Desinfektion der Flächen verwenden läßt.

Die Erfindung bezieht sich auf ein Reinigungs- und Desinfektionsmittel, das ein tertiäres Alkylamin und ein Aminoxid in einem Gewichtsverhältnis zwischen 0,2 und 2 enthält.

Bei der Wirksubstanz handelt es sich erfindungsgemäß um ein Alkylamin, das eine bakterizide, fungizide und viruzide Wirkung besitzt. Diese Substanz wird durch das Vorhandensein von Interferenzstoffen in nur äußerst geringem Maße gehemmt. Außerdem ist sie in toxikologischer und ökologischer Hinsicht völlig ungefährlich. Es wurde festgestellt, daß es vorteilhaft ist, eine synergetisch wirkende Menge eines Aminoxids mit dem vorgenannten Alkylamin zu kombinieren, um in Verbindung mit einer gleichzeitigen reinigenden Wirkung eine erhebliche Steigerung der antimikrobiellen Wirkung zu erzielen. Bei einer bevorzugten Ausführungsart der Erfindung und im Hinblick auf eine maximale Wirksamkeit entfällt auf das tertiäre Alkylamin ein Gewichtsanteil von 2 bis 15 %, vorzugsweise 2 bis 9 %, bezogen auf die gesamte Zusammensetzung. Bei dem tertiären Alkylamin handelt es sich vorzugsweise um eine Verbindung mit der allgemeinen Formel: 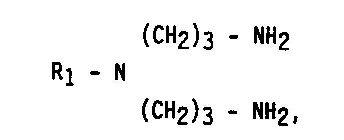 wobei R₁ für ein Alkylradikal mit 8 bis 16, vorzugsweise 10 bis 14 Kohlenstoffatomen, steht. Bei dem Aminoxid handelt es sich vorzugsweise um eine Verbindung mit der allgemeinen Formel: 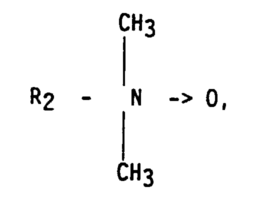 wobei R₂ für ein Alkylradikal, mit 8 bis 16, vorzugsweise 10 bis 14 Kohlenstoffatomen, steht.

Es wurde nachgewiesen, daß sich die besten Ergebnisse erzielen lassen, wenn die Radikale R₁ und R₂ identisch sind, insbesondere wenn R₁ oder R₂ ein Laurylradikal ist. In diesem Falle handelt es sich bei dem Alkylamin um Diaminopropyllaurylamin und bei dem Aminoxid um Dimethyl-laurylaminoxid.

Die erfindungsgemäße Zusammensetzung kann außerdem ein nichtionisches Tensid, das ein geringes Schäumvermögen besitzt, mit einem Gewichtsanteil von 2 bis 20 %, vorzugsweise 2 bis 13 %, bezogen auf die gesamte Zusammensetzung, sowie einen Duftstoff mit einem Gewichtsanteil von 0,005 bis 1 %, vorzugsweise 0,05 bis 0,6 %, bezogen auf die gesamte Zusammensetzung, enthalten. Bei dem nichtionischen Tensid kann es sich um ein aliphatisches Polyalkoxylderivat handeln, etwa ein Fettalkoholäthylenoxidpropylenoxid. Es wird vorzugsweise in einem Gewichtsverhältnis von 0,2 bis 4 zu dem Alkylamin bzw. dem Aminoxid benutzt. Eine bevorzugte erfindungsgemäße Zusammensetzung, bei der eine hohe Wirksamkeit festgestellt wurde, lautet wie folgt:

| | |
|---|---|
| Diaminopropyllaurylamin | 5,4 % |
| Dimethyllaurylaminoxid | 6 % |
| Polyalkoxylderivat | 8 % |
| Duftstoff | 0,1 % |
| wäßrige Hilfsstoffe | QSP 100 |

Die vorstehende Zusammensetzung wird in einem Mischer hergestellt, in den in der angegebenen Reihenfolge und unter Schütteln das gereinigte Wasser (Aqua purificata), das Dimethyllaurylaminoxid, das Diaminopropyllaurylamin und das Polyalkoxylderivat eingefüllt werden.

Für den Endgebrauch erfolgt eine Verdünnung mit Wasser im Verhältnis 20 ml Lösung auf 8 Liter Wasser, was einer Gebrauchskonzentration von 0,25 % entspricht. Das erfindungsgemäße Produkt ist eine klare, bernsteinfarbene Lösung mit einem pH-Wert von 11,2 ± 0,5. Die Abfüllung kann beispielsweise entweder in 20 ml-Beuteln oder in 5 Liter-Kanistern erfolgen. Aufgrund seiner antiseptischen Wirkung und seiner Nichttoxizität unter Beachtung der AFNOR-Normen, auf die weiter unten noch näher eingegangen wird, besteht die Möglichkeit, die erfindungsgemäße Zusammensetzung zur antiseptischen Behandlung gesunder Haut zu verwenden.

Die antimikrobielle Wirkung der bevorzugten erfindungsgemäßen Zusammensetzung (oben angeführtes Beispiel) ist entsprechend den AFNOR-Normen nachgewiesen worden. Die als Anlage beigefügten Ergebnisse beziehen sich auf die verschiedenen Tests, die nach dem Membranfilterverfahren durchgeführt wurden. Bei diesen Tests ging es um die folgenden Bestimmungsvorgänge:
- Bestimmung der bakteriziden Wirkung gemäß der Norm AFNORM NF T 72-151 (Anhang 1),
- Bestimmung der bakteriziden Wirkung in Gegenwart einer Interferenzsubstanz (hartes Wasser) gemäß der Norm NF T 72-171 (Anhang 2),
- Bestimmung der bakteriziden Wirkung in Gegenwart einer Interferenzsubstanz (Albumin) gemäß der Norm NF T 72-171 (Anhang 3),
- Bestimmung der fungiziden Wirkung gemäß der Norm NF T 72-201 (Anhang 4).

Die Bestimmung der bakteriziden Wirkung gegenüber Mycobacterium Tuberculosis nach dem Bactec TB-Verfahren wurde ebenfalls durchgeführt. Bei dem Bactec TB-Medium (12B) handelt es sich um einen Nährboden auf der Basis von angereichertem Middelbrook 7H9. Die Mykobakterien benutzen eine in dem Medium vorhandene, mit Kohlenstoff-14 markierte Fettsäure und geben CO₂ an die Luft ab.

Wenn ein Mykobakterienwachstum in den 12B-Flaschen vorliegt, mißt der Bactec 460 die durch das freigesetzte 14CO₂ bedingte Radioaktivität und stellt sie numerisch auf einer Skala von 0 bis 999 dar. Diese Werte werden als Wachstumsindex (GI) bezeichnet. Die Vergrößerung des GI ist proportional zu der Bakterienzahl. Nach der Einwirkung des Desinfektionsmittels auf die Mycobacterium Tuberculosis-Suspension erfolgt die Auszählung der verbliebenen Mykobakterien durch Injektion jeder Keimsuspensionsverdünnung in 2 12B-Flaschen für mehrere Verdünnungen. Die Erhöhung des GI entspricht dem Vorhandensein von Mykobakterien.

Die Methode besteht in der Verwendung einer McFarland 3-Suspension in sterilem destilliertem Wasser, die aus einer Reinkultur von Mycobacterium Tuberculosis zubereitet wird, die aus einem Löwenstein-Nährboden entnommen wird. Anhand dieser "Mutter"-Lösung werden fortschreitend Verdünnungen um Faktor 10 bis zu 10⁻⁹ hergestellt. 100 µl jeder Verdünnung werden in ein 12B-Medium verimpft, das 3 ml Kulturnährboden enthält (Verdünnung 1/40 in jeder 12B-Flasche). Anschließend werden 200 µl reines oder verdünntes Desinfektionsmittel mit 200 µl Mycobacterium Tuberculosis mit McFarland 3 über einen bestimmten Zeitraum unter Schütteln in einem 35 ml-Zentrifugenröhrchen in Kontakt gebracht. Nach Ablauf der Kontaktzeit wird das Röhrchen mit sterilem destilliertem Wasser befüllt und anschließend 30 Minuten lang mit 3000 U/min. zentrifugiert. Der Überstand wird abgehoben, und der Rückstand wird in 2 ml destilliertem Wasser aufgenommen (Verdünnung 1/10 bis zu 10⁻⁵). Anschließend wird die Substanz in 2 12B-Flaschen eingefüllt, die 4 ml Nährlösung enthalten:
100 µl der Verdünnung 10⁻¹ (Verdünnung 1/40)
100 µl der Verdünnung 10⁻²
100 µl der Verdünnung 10⁻³
100 µl der Verdünnung 10⁻⁴
100 µl der Verdünnung 10⁻⁵

Das Ablesen der Auszählung und des Versuchs erfolgt am Bactec-TB-Leser. Die als GI angegebenen Resultate sind aus den in Anhang 5 für die einzelnen Kontaktzeiten angeführten Tabellen zu entnehmen, wobei das Ablesen nach einer Doppel-Beimpfung der 12B-Nährböden zum Zeitpunkt T1, T2, T3, T6, T7, T8, T10, T13, T15, T16, T17, T20 erfolgt. Die an der bevorzugten erfindungsgemäßen Zusammensetzung nach der vorstehenden Definition durchgeführten toxikologischen Untersuchungen führten zu den folgenden Ergebnissen:
a) akute Toxizität bei der männlichen Maus nach oraler Verabreichung
   - LD50 > 2,5 ml/kg
b) Index der akuten Augenreizung
   - 9,3 bis 24 Stunden (Produkt mit schwacher Reizwirkung)
c) Index der primären Hautreizung
   - 0,71 (Produkt mit schwacher Reizwirkung)
d) Index der primären Hautreizung des Produkts in der Gebrauchskonzentration (Verdünnung auf 0,25 %)
   - 0,25 (Produkt ohne Reizwirkung)

### Beispiele

### Anhang 1

Bestimmung der bakteriziden Wirkung:
Norm AFNOR T 72-151
Stand: November 1987

| I. Angaben zu den Proben:: | |
|---|---|
| Produktbezeichnung und Chargennummer | Reinigungs- und Desinfektionsmittel für Böden und Flächen |
| Hersteller | PARAGERM |
| Analysezeitraum | Mai 1993 |
| Wirksubstanzen und Konzentrationen | auf vertraulicher Basis mitgeteilt |

| II. Versuchsbedingungen:: | |
|---|---|
| Versuchstemperatur | 20°C |
| Bei den Versuchen benutztes Verdünnungsmittel | Steriles destilliertes Wasser |

| III. Im Anschluß an den Vorversuch bestimmte Vorgehensweise:: | | |
|---|---|---|
| Art der Membranen und Kenndaten | | Millipore-Filter Typ HA, Porengröße 0,45 µ |
| Spülflüssigkeit | Zusammensetzung | |
| | - Polysorbat | 3% |
| | - Lecithin | 0,3% |
| | - Histidin | 0,1% |
| | - Natriumthiosulfat | 0,5% |
| Anzahl der Spülungen mit dieser Flüssigkeit : | | 3 |
| Für jede Spülung benutztes Volumen : | | 50 ml |

Dem Auszählmedium zugesetztes Neutralisationsmittel und Konzentration:

| Neutralisierender Nährboden für die Auszählung | |
|---|---|
| Zusammensetzung: | |
| Rindfleischextrakt | 3 g |
| Tryptisches Caseinpepton | 5 g |
| Glucose | 1 g |
| Lecithin | 1 g |
| Polysorbat | 7 g |
| Agar-Agar in Pulverform | 15 g |
| Destilliertes Wasser | 1000 ml |

Bestimmung der bakteriziden Wirkung des Produkts:
SPEKTRUM 4 0 SPEKTRUM 5 X

| Ergebnisse der Vorversuche unter den beschriebenen Bedingungen | | | | |
|---|---|---|---|---|
| Getestete Konzentrationen des Produkts (V/V) | Stämme, Herkunftssammlung und Nummer in der Sammlung | Ausgezählte Kolonien | | |
| | | N | N' | n |
| 0,125 % | Pseudomonas aeruginosa CIP A 22 | 163 | 153 | 121 |
| | | | | |
| 0,25 % | Escherichia coli CIP 54 127 | 125 | 84 | 8 |
| | | | | |
| 0,25 % | Staphylococcus aureus CIP 53 154 | 132 | 124 | 130 |
| | | | | |
| 0,25 % | Enterococcus hirae CIP 5 855 SPEKTRUM 4 | 85 | 94 | 65 |
| | | | | |
| 0,25 % | Mycobacterium smegmatis CIP 7 326 SPEKTRUM 5 | 127 | 75 | 70 |

| Ergebnisse der eigentlichen Versuche unter den beschriebenen Bedingungen | | | | | | | | |
|---|---|---|---|---|---|---|---|---|
| **STÄMME** | N | N' 50 bis 150 | n | pH | | ausgezählte Kolonien ( + = mehr als 150 Kolonien) Konzentration d. Produkts in Kontakt mit mit d. Bakterien (% V/V) | | |
| | | | | Min. | max. | 0,06 % | 0,125 % | 0,25 % |
| *Pseudomonas aeruginosa | 163 | 151 | 121 | 6,10 | 6,40 | 2 | 0 | |
| | | | | | | | | |
| *Escherichia coli | 125 | 84 | 80 | 6,10 | 6,40 | 12 | 1 | - |
| *Staphylococcus aureus | 132 | 124 | 30 | 6,10 | 6,40 | 0 | 0 | 0 |
| | | | | | | | | |
| *Enterococcus hirae SPEKTRUM 4 | 85 | 94 | 65 | 6,10 | 6,40 | 0 | 0 | 0 |
| | | | | | | | | |
| *Mycobacterium smegmatis SPEKTRUM 5 | 127 | 75 | 70 | 6,10 | 6,40 | | 0 | 0 |

Bakterizide Konzentration des getesteten Produkts in bezug auf:

| | |
|---|---|
| Pseudomonas aeruginosa | ≤ 0,06% |
| Escherichia coli | ≤ 0,06% |
| Staphylococcus aureus | ≤ 0,06% |
| Enterococcus hirae | ≤ 0,06% |
| Mycobacterium smegmatis | ≤ 0,125% |

Bezogen auf die Gesamtheit der getesteten Stämme entspricht das Produkt der Norm T 72-151 in der Konzentration von: 0,125 %

### Anhang 2

Bestimmung der bakteriziden Wirkung in Gegenwart von Interferenzsubstanzen Norm AFNOR T 72-171

| I. Angaben zu den Proben:: | |
|---|---|
| Produktbezeichnung und Chargennummer | Reinigungs- und Desinfektionsmittel für Böden und Flächen |
| Hersteller | PARAGERM |
| Analysezeitraum | Mai 1993 |
| Wirksubstanzen und Konzentrationen | auf vertraulicher Basis mitgeteilt |

| II. Versuchsbedingungen:: | |
|---|---|
| Versuchstemperatur | 20°C |
| Bei den Versuchen benutztes Verdünnungsmittel | Steriles destilliertes Wasser |
| Interferenzsubstanzen | Hartes Wasser |

| III. Im Anschluß an den Vorversuch bestimmte Vorgehensweise:: | | |
|---|---|---|
| Art der Membranen und Kenndaten | | Millipore-Filter Typ HA, Porengröße 0,45 µ |
| Spülflüssigkeit | Zusammensetzung | |
| | - Polysorbat | 3% |
| | - Lecithin | 0,3% |
| | - Histidin | 0,1% |
| | - Natriumthiosulfat | 0,5% |
| Anzahl der Spülungen mit dieser Flüssigkeit | | 3 |
| Für jede Spülung benutztes Volumen | | 50 ml |

Dem Auszählmedium zugesetztes Neutralisationsmittel und Konzentration:

| Neutralisierender Nährboden für die Auszählung | |
|---|---|
| Zusammensetzung: | |
| Rindfleischextrakt | 3 g |
| Tryptisches Caseinpepton | 5 g |
| Glucose | 1 g |
| Lecithin | 1 g |
| Polysorbat | 7 g |
| Agar-Agar in Pulverform | 15 g |
| Destilliertes Wasser | 1000 ml |

Bestimmung der bakteriziden Wirkung des Produkts:
SPEKTRUM 4 X SPEKTRUM 5 0

| Ergebnisse der Vorversuche unter den beschriebenen Bedingungen: | | | | |
|---|---|---|---|---|
| Getestete Konzentrationen des Produkts (V/V) | Stämme, Herkunftssammlung und Nummer in der Sammlung | Ausgezählte Kolonien | | |
| | | N | N' | n |
| 0,5 % | Pseudomonas aeruginosa CIP A 22 | 62 | 61 | 58 |
| | | | | |
| 0,25 % | Escherichia coli CIP 54 127 | 147 | 126 | 108 |
| | | | | |
| 0,25 % | Staphylococcus aureus CIP 53 154 | 97 | 104 | 100 |
| | | | | |
| 0,5 % | Enterococcus hirae CIP 5 855 SPEKTRUM 4 | 72 | 92 | 89 |
| | | | | |
| | Mycobacterium smegmatis CIP 7 326 SPEKTRUM 5 | | | |

| Ergebnisse der eigentlichen Versuche unter den beschriebenen Bedingungen | | | | | | | | |
|---|---|---|---|---|---|---|---|---|
| **STÄMME** | N | N' 50 bis 150 | n | pH | | ausgezählte Kolonien (+ = mehr als 150 Kolonien) Konzentration d. Produkts in Kontakt m. d. Bakt.(%V/V) | | |
| | | | | min. | max. | 0,125% | 0,25% | 0,5% |
| *Pseudomonas aeruginosa | 62 | 61 | 58 | 6,20 | 8,10 | | + | 53 |
| | | | | | | | | |
| *Escherichia coli | 147 | 126 | 108 | 6,40 | 6,20 | 0 | 0 | |
| | | | | | | | | |
| *Staphylococcus aureus | 97 | 104 | 100 | 6,40 | 6,70 | 0 | 0 | |
| | | | | | | | | |
| *Enterococcus hirae SPEKTRUM 4 | 72 | 92 | 89 | 6,70 | 8,10 | 0 | 0 | 0 |
| | | | | | | | | |
| *Mycobacterium smegmatis SPEKTRUM 5 | | | | | | | | |

Bakterizide Konzentration des getesteten Produkts in bezug auf:

| | |
|---|---|
| Pseudomonas aeruginosa | ≤ 0,5% |
| Escherichia coli | ≤ 0,125% |
| Staphylococcus aureus | ≤ 0,125% |
| Enterococcus hirae | ≤ 0,125% |
| | |
| Mycobacterium smegmatis | ≤ |

Bezogen auf die Gesamtheit der getesteten Stämme entspricht das Produkt der Norm T 72-171 in der Konzentration von: 0,5% in Gegenwart von hartem Wasser

### Anhang 3

Bestimmung der bakteriziden Wirkung in Gegenwart von Interferenzsubstanzen Norm AFNOR T 72-171

| I. Angaben zu den Proben: | |
|---|---|
| Produktbezeichnung und Chargennummer | Reinigungs- und Desinfektionsmittel für Böden und Flächen |
| Hersteller | PARAGERM |
| Analysezeitraum | Mai 1993 |
| Wirksubstanzen und Konzentrationen | auf vertraulicher Basis mitgeteilt |

| II. Versuchsbedingungen: | |
|---|---|
| Versuchstemperatur | 20°C |
| Bei den Versuchen benutztes Verdünnungsmittel | Steriles destilliertes Wasser |
| Interferenzsubstanzen | Albumin - Hefeextrakt |

| III. Im Anschluß an den Vorversuch bestimmte Vorgehensweise: | | |
|---|---|---|
| Art der Membranen und Kenndaten | | Millipore-Filter Typ HA, Porengröße 0,45 µ |
| Spülflüssigkeit | Zusammensetzung | |
| | - Polysorbat | 3% |
| | - Lecithin | 0,3% |
| | - Histidin | 0,1% |
| | - Natriumthiosulfat | 0,5% |
| Anzahl der Spülungen mit dieser Flüssigkeit | | 3 |
| Für jede Spülung benutztes Volumen | | 50 ml |

Dem Auszählmedium zugesetztes Neutralisationsmittel und Konzentration:

| Neutralisierender Nährboden für die Auszählung | |
|---|---|
| Zusammensetzung: | |
| Rindfleischextrakt | 3 g |
| | |
| Tryptisches Caseinpepton | 5 g |
| Glucose | 1 g |
| Lecithin | 1 g |
| Polysorbat | 7 g |
| Agar-Agar in Pulverform | 15 g |
| Destilliertes Wasser | 1000 ml |

Bestimmung der bakteriziden Wirkung des Produkts:
SPEKTRUM 4 X SPEKTRUM 5 0

| Ergebnisse der eigentlichen Versuche unter den beschriebenen Bedingungen | | | | |
|---|---|---|---|---|
| Getestete Konzentrationen des Produkts (V/V) | Stämme, Herkunftssammlung und Nummer in der Sammlung | Ausgezählte Kolonien | | |
| | | N | N' | n |
| 0,5 % | Pseudomonas aeruginosa CIP A 22 | 172 | 186 | 163 |
| | | | | |
| 0,5 % | Escherichia coli CIP 54 127 | 90 | 64 | 68 |
| | | | | |
| 0,5 % | Staphylococcus aureus CIP 53 154 | 53 | 56 | 53 |
| | | | | |
| 0,5 % | Enterococcus hirae CIP 5 855 SPEKTRUM 4 | 102 | 101 | 109 |
| | | | | |
| | Mycobacterium smegmatis CIP 7 326 SPEKTRUM 5 | | | |

| Ergebnisse der eigentlichen Versuche unter den beschriebenen Bedingungen | | | | | | | |
|---|---|---|---|---|---|---|---|
| **STÄMME** | N | N' 50 bis 150 | n | pH | | ausgezählte Kolonien (+ = mehr als 150 Kolonien) Konzentration d. Produkts in Kontakt m. d. Bakt. (%V/V) | |
| | | | | min. | max. | 0,25% | 0,5% |
| *Pseudomonas aeruginosa | 172 | 186 | 163 | | 6,70 | | 8 |
| | | | | | | | |
| *Escherichia | | | | | | | |
| | | | | | | | |
| coli | 90 | 64 | 68 | | 6,70 | | 0 |
| | | | | | | | |
| *Staphylococcus | | | | | | | |
| | | | | | | | |
| aureus | 53 | 56 | 53 | | 6,70 | | 26 |
| | | | | | | | |
| *Enterococcus hirae SPEKTRUM 4 | 102 | 101 | 109 | | 6,70 | | 95 |
| | | | | | | | |
| *Mycobacterium smegmatis SPEKTRUM 5 | | | | | | | |

Bakterizide Konzentration des getesteten Produkts in bezug auf:

| | |
|---|---|
| Pseudomonas aeruginosa | ≤ 0,5 % |
| Escherichia coli | ≤ 0,5 % |
| Staphylococcus aureus | ≤ 0,5 % |
| | |
| Mycobacterium smegmatis | ≤ |

Bezogen auf die Gesamtheit der getesteten Stämme entspricht das Produkt der Norm T 72 - 171 in der Konzentration von: 0,5 %

### Anhang 4

Bestimmung der fungiziden Wirkung
Norm AFNOR T 72-201
Stand: September 1987

| I. Angaben zu den Proben: | |
|---|---|
| Produktbezeichnung und Chargennummer | Reinigungs- und Desinfektionsmittel für Böden und Flächen Filtration |
| Hersteller | PARAGERM |
| Analysezeitraum | Mai 1993 |
| Wirkstubstanzen und Konzentration | auf vertraulicher Basis mitgeteilt |

| II. Versuchsbedingungen: | |
|---|---|
| Versuchstemperatur | 20 °C |
| Bei den Versuchen benutztes Verdünnungsmittel | Steriles destilliertes Wasser |

| III. Im Anschluß an den Vorversuch bestimmte Vorgehensweise:: | | |
|---|---|---|
| Art der Membranen und Kenndaten | | Millipore-Filter Typ HA, Porengröße 0,45 µ |
| Spülflüssigkeit | Zusammensetzung | |
| | - Polysorbat | 3% |
| | - Lecithin | 0,3% |
| | - Histidin | 0,1% |
| | - Natriumthiosulfat | 0,5% |
| | | |
| Anzahl der Spülungen mit dieser Flüssigkeit | | 3 |
| Für jede Spülung benutztes Volumen | | 50 ml |

Dem Auszählmedium zugesetztes Neutralisationsmittel und Konzentration: Entfällt

| Ergebnisse der Vorversuche unter den beschriebenen Bedingungen | | | | |
|---|---|---|---|---|
| Getestete Konzentrationen des Produkts (V/V) | Stämme, Herkunftssammlung und Nummer in der Sammlung | Ausgezählte Kolonien | | |
| | | N | N' | n |
| | Penicillinum verrucosum var. cyclopium IP 1231-80 = LCP 282 | | | |
| | | | | |
| | Cladosporium cladosporioides IP 1232-80 = LCP 484 | | | |
| | | | | |
| | Absidia corymbifera IP 1129-75 = LCP 1942 | | | |
| | | | | |
| 0,5 % | Candida albicans IP 1180-79 = LCP 409 =ATCC 2091 | 111 | 108 | 95 |

| Ergebnisse der eigentlichen Versuche unter den beschriebenen Bedingungen | | | | | | | |
|---|---|---|---|---|---|---|---|
| **STÄMME** | N | N' | n | pH | | ausgezählte Kolonien (+ = mehr als 200 Kolonien) Konzentration d. Produkts in Kontakt m. d. Bakt.(%V/V) | |
| | | | | min. | max. | 0,06% | 0,125% |
| *Penicillinum verrucosum | | | | | | | |
| | | | | | | | |
| *Cladosporium cladosporioides | | | | | | | |
| | | | | | | | |
| *Absidia corymbifera | | | | | | | |
| | | | | | | | |
| *Candida albicans | 111 | 108 | 95 | 6,10 | 6,40 | 0 | 0 |

Fungizide Konzentration des getesteten Produkts in bezug auf:

| | |
|---|---|
| Penicillinum verrucosum | % |
| Cladosporium cladosporioides | % |
| Absidia crymbifera | % |
| Candida albicans | ≤0,6% |

### Anhang 5

| | | | | | | | | | | | | |
|---|---|---|---|---|---|---|---|---|---|---|---|---|
| STAMM : M. Tuberculosis getestet ohne Desinfektionsmittel | | | | | | | | | | | | |
| Aus einer McFarland 3-Suspension des Stammes werden 100 µl jeder Verdünnung (10⁻¹ bis 10⁻⁵) in eine 12B-Flasche verimpft. | | | | | | | | | | | | |

| | T1 12. Mai | T2 13. Mai | T3 14. Mai | T6 17. Mai | T7 18. Mai | T8 19. Mai | T10 21. Mai | T13 24. Mai | T15 26. Mai | T16 27. Mai | T17 28. Mai | T21 1. Juli |
|---|---|---|---|---|---|---|---|---|---|---|---|---|
| - 1 | 14 | 64 | 109 | 999 | gerein. | - | - | - | - | - | - | - |
| - 2 | 1 | 8 | 14 | 175 | 204 | 314 | 999 | gerein. | - | - | - | - |
| - 3 | 0 | 0 | 0 | 4 | 8 | 19 | 148 | 584 | 750 | 810 | 890 | 999 |
| - 4 | 0 | 0 | 0 | 0 | 0 | 1 | 18 | 84 | 154 | 170 | 261 | 595 |
| - 5 | 0 | 0 | 0 | 0 | 0 | 0 | 0 | 1 | 4 | 4 | 8 | 29 |

| Desinfektionsmittel für Böden und Flächen, verdünnt auf 0,25%, getestet bei 50% mit M. Tuberculosis Kontaktzeit : 5 Minuten unter Schütteln AK | | | | | | | | | | | | |
|---|---|---|---|---|---|---|---|---|---|---|---|---|
| | T1 12. Mai | T2 13. Mai | T3 14. Mai | T6 17. Mai | T7 18. Mai | T8 19. Mai | T10 21. Mai | T13 24. Mai | T15 26. Mai | T16 27. Mai | T17 28. Mai | T21 1. Juli |
| - 1 | 0 | 0 | 0 | 68 | 88 | 124 | 398 | 999 | gerein. | - | - | - |
| - 2 | 0 | 0 | 0 | 0 | 4 | 4 | 29 | 120 | 188 | 250 | 298 | 815 |
| - 3 | 4 | 0 | 0 | 0 | 0 | 0 | 8 | 50 | 135 | 175 | 324 | 885 |
| - 4 | 0 | 0 | 0 | 0 | 0 | 0 | 0 | 4 | 4 | 4 | 5 | 18 |
| - 5 | 0 | 0 | 0 | 0 | 0 | 0 | 0 | 0 | 0 | 0 | 0 | 0 |
| Der ohne Desinfektionsmittel getestete M-Tuberculosis-Stamm wuchs bis 10⁻⁵ nach 15 Tagen Kultur. In Gegenwart des 0,25%-igen Desinfektionsmittels wird ein Wachstum bei 10⁻⁴ nach 13 Tagen Kultur festgestellt. Kein Wachstum an den ersten Tagen bei der Verdünnung 10⁻¹. | | | | | | | | | | | | |

| Desinfektionsmittel für Boden und Flächen, verdünnt auf 0,55%, getestet bei 50% mit M. Tuberculosis Kontaktzeit : 5 Minuten unter Schütteln AM | | | | | | | | | | | | |
|---|---|---|---|---|---|---|---|---|---|---|---|---|
| | T1 12. Mai | T2 13. Mai | T3 14. Mai | T6 17. Mai | T7 18. Mai | T8 19. Mai | T10 21. Mai | T13 24. Mai | T15 26. Mai | T16 27. Mai | T17 28. Mai | T21 1. Juli |
| - 1 | 0 | 0 | 0 | 74 | 94 | 135 | 474 | 999 | gerein. | - | - | - |
| - 2 | 0 | 0 | 0 | 8 | 10 | 14 | 54 | 288 | 398 | 540 | 621 | 999 |
| - 3 | 0 | 0 | 0 | 0 | 0 | 0 | 1 | 18 | 24 | 28 | 34 | 84 |
| - 4 | 0 | 0 | 0 | 0 | 0 | 0 | 0 | 11 | 18 | 21 | 24 | 68 |
| - 5 | 0 | 0 | 0 | 0 | 0 | 0 | 0 | 0 | 0 | 0 | 0 | 0 |
| Es ist eine ähnliche Entwicklung wie in der vorangehenden Tabelle festzustellen. | | | | | | | | | | | | |

## Patentansprüche

1. Reinigungs- und Desinfektionsmittel, dadurch gekennzeichnet, daß es ein tertiäres Alkylamin der Formel 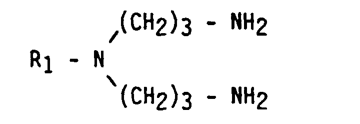 und ein Aminoxid der Formel 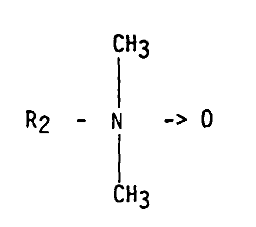 enthält, wobei R₁ und R₂ Alkylradikale mit 8 bis 16, vorzugsweise 10 bis 14 Kohlenstoffatomen sind und das Gewichtsverhältnis zwischen dem tertiären Alkylamin und dem Aminoxid zwischen 0,2 und 2 liegt.

2. Reinigungs- und Desinfektionsmittel nach Anspruch 1, bei dem die Radikale R₁ und R₂ identisch sind.

3. Reinigungs- und Desinfektionsmittel nach Anspruch 2, bei dem das mit R₂ identische R₁ ein Laurylradikal ist, während es sich bei dem tertiären Alkylamin um Diaminopropyllaurylamin und bei dem Aminoxid um Dimethyllaurylaminoxid handelt.

4. Reinigungs- und Desinfektionsmittel nach Anspruch 3, das 2 bis 15 Gew.-% Diaminopropyllaurylamin, bezogen auf die gesamte Zusammensetzung enthält.

5. Reinigungs- und Desinfektionsmittel nach einem der vorangehenden Ansprüche, das außerdem ein nichtionisches Tensid enthält.

6. Reinigungs- und Desinfektionsmittel nach Anspruch 5, bei dem das nichtionische Tensid ein aliphatisches Polyalkoxylderivat ist.

7. Reinigungs- und Desinfektionsmittel 1 nach Anspruch 6, bei dem das nichtionische Tensid ein Fettalkoholäthylenoxidpropylenoxid ist.

8. Reinigungs- und Desinfektionsmittel nach einem der Ansprüche 5 bis 7, bei dem das nichtionische Tensid in einem Gewichtsverhältnis von 0,2 bis 4 zu dem tertiären Alkylamin bzw. dem Aminoxid enthalten ist.

9. Reinigungs- und Desinfektionsmittel nach einem der vorangehenden Ansprüche, das außerdem einen Duftstoff enthält.

10. Reinigungs- und Desinfektionsmittel nach einem der Ansprüche 5 bis 9, das 2 bis 20 Gew.-% nichtionische Tensid und 0,05 bis 1 Gew.-% Duftstoff, bezogen auf die gesamte Zusammensetzung, enthält.

11. Reinigungs- und Desinfektionsmittel nach einem der vorangehenden Ansprüche, das die folgenden Bestandteile, verdünnt in Wasser, enthält:
| | |
|---|---|
| Diaminopropyllaurylamin | 5,4 Gew.-% |
| Dimethyllaurylaminoxid | 6 Gew.-% |
| Polyalkoxylderivat | 8 Gew.-% |
| Duftstoff | 0,1 Gew.-% |

12. Reinigungs- und Desinfektionslösung nach einem der Ansprüche 1 bis 11, insbesondere zur Behandlung von Böden und Wände in Krankenhäusern.

13. Anwendung des Reinigungs- und Defektionsmittels nach einem der Ansprüche 1 bis 11, insbesondere zur antiseptischen Behandlung der menschlichen Haut.

## Claims

1. A cleaning and disinfecting formulation, characterized in that it contains a tertiary alkylamine corresponding to the following formula: 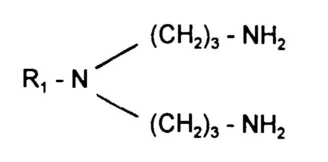 and an amine oxide corresponding to the following formula: 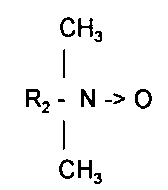 in which R₁ and R₂ are alkyl radicals containing 8 to 16 and preferably 10 to 14 carbon atoms,
and in that the ratio by weight between the tertiary alkylamine and the amine oxide is between 0.2 and 2:1.

2. A cleaning and disinfecting formulation as claimed in claim 1, in which the radicals R₁ and R₂ are identical.

3. A cleaning and disinfecting formulation as claimed in claim 2, in which the R₁ identical with R₂ is a lauryl radical while the tertiary alkylamine is diaminopropyl laurylamine and the amine oxide is dimethyl laurylamine.

4. A cleaning and disinfecting formulation as claimed in claim 3 containing 2 to 15% by weight of diaminopropyl laurylamine, based on the composition as a whole.

5. A cleaning and disinfecting formulation as claimed in any of the preceding claims additionally containing a nonionic surfactant.

6. A cleaning and disinfecting formulation as claimed in claim 5, in which the nonionic surfactant is an aliphatic polyalkoxyl derivative.

7. A cleaning and disinfecting formulation as claimed in claim 6, in which the nonionic surfactant is a fatty alcohol ethylene oxide/propylene oxide.

8. A cleaning and disinfecting formulation as claimed in any of claims 5 to 7, in which the nonionic surfactant is present in a ratio by weight of 0.2 to 4:1 to the tertiary alkylamine or the amine oxide.

9. A cleaning and disinfecting formulation as claimed in any of the preceding claims which additionally contains a fragrance.

10. A cleaning and disinfecting formulation as claimed in any of claims 5 to 9 which contains 2 to 20% by weight of nonionic surfactant and 0.05 to 1% by weight of fragrance, based on the composition as a whole.

11. A cleaning and disinfecting formulation as claimed in any of the preceding claims which contains the following constituents diluted with water:
| | |
|---|---|
| diaminopropyl laurylamine | 5.4% by weight |
| dimethyl laurylamine oxide | 6 % by weight |
| polyalkoxyl derivative | 8 % by weight |
| fragrance | 0.1% by weight. |

12. A cleaning and disinfecting solution as claimed in any of claims 1 to 11, more particularly for treating floors and walls in hospitals.

13. The use of the cleaning and disinfecting formulation claimed in any of claims 1 to 11, more particularly for the antiseptic treatment of human skin.

## Revendications

1. Produit de nettoyage et de désinfection caractérisé en ce qu'
il renferme une alkylamine tertiaire de formule : 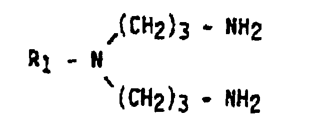 et un oxyde d'amine de formule : 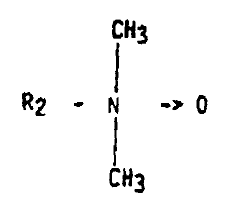 dans lesquelles R₁ et R₂ sont des radicaux alkyle ayant de 8 à 16, de préférence de 10 à 14 atomes de carbone et pour lequel le rapport pondéral entre l'alkylamine tertiaire et l'oxyde d'amine se situe entre 0,2 et 2.

2. Produit de nettoyage et de désinfection selon la revendication 1,
dans lequel les radicaux R₁ et R₂ sont identiques.

3. Produit de nettoyage et de désinfection selon la revendication 2, dans lequel le radical R₁ identique à R₂ est un radical lauryle, tandis qu'il s'agit pour l'amine tertiaire d'une diaminopropyllaurylamine et pour l'oxyde d'amine d'un oxyde de diméthyllaurylamine.

4. Produit de nettoyage et de désinfection selon la revendication 3,
contenant de 2 à 15 % en poids de diaminopropyllaurylamine rapporté à la composition totale.

5. Produit de nettoyage et de désinfection selon l'une des revendications précédentes,
contenant en outre un agent tensioactif non ionique.

6. Produit de nettoyage et de désinfection selon la revendication 5,
où l'agent tensioactif est un dérivé polyalkoxylé aliphatique.

7. Produit de nettoyage et de désinfection selon la revendication 6,
où l'agent tensioactif non ionique est un alcool gras/oxyde d'éthylène/oxyde de propylène.

8. Produit de nettoyage et de désinfection selon l'une des revendications 5 à 7,
où l'agent tensioactif non ionique est contenu dans un rapport en poids de 0,2 à 4 par rapport à l'alkylamine tertiaire ou à l'oxyde d'amine.

9. Produit de nettoyage et de désinfection selon l'une des revendications précédentes,
renfermant en outre un parfum.

10. Produit de nettoyage et de désinfection selon l'une des revendications 5 à 9,
renfermant de 2 à 20 % en poids d'agent tensioactif non ionique et de 0,05 à 1 % en poids de parfum, rapporté à la composition totale.

11. Produit de nettoyage et de désinfection selon l'une des revendications précédentes,
renfermant les constituants suivants dilués dans l'eau :
| | |
|---|---|
| Diaminopropyllaurylamine | 5,4 % en poids |
| Diméthyllaurylamine | 6 % en poids |
| dérivé polyalkoxylé | 8 % en poids |
| Parfum | 0,1 % en poids. |

12. Solution de produit de nettoyage et de désinfection selon l'une des revendications 1 à 11,
en particulier pour le traitement des sols et des murs dans les hôpitaux.

13. Utilisation du produit de nettoyage et de désinfection selon l'une des revendications 1 à 11,
en particulier pour le traitement antiseptique de la peau chez l'homme.
